(19) Europäisches Patentamt
European Patent Office
Office européen des brevets

(11) Publication number: 0 311 377 A2

# (12) EUROPEAN PATENT APPLICATION

(21) Application number: 88309282.7

(22) Date of filing: 05.10.88

(51) Int. Cl.[4]: C 12 M 1/40
G 01 N 27/30, G 01 N 27/46
// C12Q1/32, C12Q1/54

(30) Priority: 05.10.87 US 104862

(43) Date of publication of application:
12.04.89 Bulletin 89/15

(84) Designated Contracting States:
AT BE CH DE ES FR GB IT LI LU NL SE

(71) Applicant: ARDEN MEDICAL SYSTEMS, INC.
2675 Long Lake Road
Roseville Minnesota 55113 (US)

(72) Inventor: Anderson, Carter
6057 67th Way
Brooklyn Park, MN 55417 (US)

Sogin, David C.
2654 Irving Avenue South
Minneapolis, MN 55408 (US)

Fowler, William V.
4925 Nokomis Avenue South
Minneapolis, MN 55417 (US)

(74) Representative: Mercer, Christopher Paul et al
Carpmaels & Ransford 43, Bloomsbury Square
London WC1A 2RA (GB)

The title of the invention has been amended (Guidelines for Examination in the EPO, A-III, 7.3).

## (54) Sensor for measurement of a chemical species susceptible to dehydrogenation.

(57) An electrode is provided for a single-use sensing device in a clinical chemical analyzer for amperometrically determining the level, in a body fluid, of a chemical species susceptible to dehydrogenation in the presence of a dehydrogenase enzyme. The electrode has a coating comprising the dehydrogenase enzyme, a perfluorosulfonic acid polymer, methylene blue and a cofactor of the group consisting of $NAD^+$ and $NADP^+$. In a preferred embodiment, the coating also includes a water soluble resinous polymer and a cured aqueous emulsion adhesive.

In another embodiment, the sensor is used to measure a hydrogenatable chemical species, in which case the cofactor is in hydrogenated form. In still another embodiment, the sensor is used to measure a dehydrogenase enzyme, in which case the enzyme is omitted from the coating and a dehydrogenatable chemical species is substituted therefor.

EP 0 311 377 A2

## Description

## Sensor For Measurement Of Enzyme Hydrogenatable/Dehydrogenatable Chemical Species In Aqueous Solutions

REFERENCE TO COPENDING APPLICATIONS

Reference is made to copending patent applications entitled "Disposable Single-Use Sensing Device For Clinical Analyzer", Serial No. 550,313, filed November 10, 1983 by Richard L. Little and Ronald C. Laska and "Multiple Species Group Disposable Sensing Device For Clinical Chemistry Analyzer" Serial No. 550,361, filed November 13, 1983 by Mark B. Knudsen, Walter L. Sembrowich and Scott W. Carlson. These applications are assigned to the same assignee as the present application; and their disclosures are incorporated by reference.

Background of the Invention

1. Field of the Invention

The present invention relates to medical devices. In particular, it relates to clinical chemical analyzers which are used to amperometrically measure the levels of enzyme-dehydrogenatable or hydrogenatable substances, such as glucose or pyruvic acid, in aqueous solutions and particularly in body fluids, such as whole blood, blood plasma and urine.

2. Description of the Prior Art

There is a need in modern diagnostic medicine for accurate, reliable and rapid information of the levels of certain chemical and/or biological materials in the blood, or other body fluid of a patient. One of the most common determinations is the determination of glucose levels in blood or urine, and for convenience, the description hereinafter focusses on the determination of glucose levels.

A useful system for such analyses utilizing single-use sensing devices is disclosed in co-assigned U.S. Patent 4,654,127, granted to Richard W. Baker and Roger L. Funk on March 31, 1987, the disclosure of which is incorporated herein by reference.

In the device of U.S. Patent 4,654,127, a sample of a body fluid, such as blood or urine, is placed in one chamber of a multichamber reservoir while a calibrant liquid is placed in another chamber of the reservoir. Flow to a sensor electrode is then provided, first for the calibrant liquid, and then for the test sample. The successive contacts of the fluids with the sensor electrode generate electrical currents which are measured and correlated to provide the desired information concerning the concentration of a specific substance in the test fluid. The carrier is then discarded.

The sensor electrodes in the system of U.S. Patent 4,654,127 are disclosed as each having a coating which includes a polymer and an electroactive species, with the polymer serving the function of creating a membrane over the sensor active area and immobilizing the electroactive species next to the electrically conductive surface of the sensor.

It is also known that some materials, such as glucose, whose levels in solution are desired to be measured, are susceptible to dehydrogenation in the presence of an appropriate dehydrogenase enzyme and that such dehydrogenation liberates electrons and thereby creates measurable electrical currents. In most cases, the dehydrogenation is not transformed directly to an electrical current, but rather through a reaction scheme involving a cofactor, such as NADH (the reduced form of $NAD^+$ - nicotinamide adenine dinucleide), and a mediator. The general chemistry of such systems is discussed in an article by Lo Gorton entitled "Chemical Modified Electrodes for the Electrocatalytic Oxidation of Nicotinamide Coenzymes" - J. Chem. Soc. Faraday Trans. I., 1986, 82, 1245-1258, which is incorporated herein by reference. The reaction scheme in the Lo Gorton article is represented by:

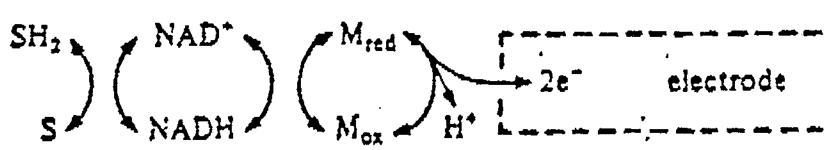


where $SH_2$ and S represent the hydrogenated and dehydrogenated forms, respectively, of the species whose concentration is to be determined and $M_{ox}$ and $M_{red}$ represent the oxidized and reduced forms of the mediator, respectively.

The most common assay system for the electrochemical detection of glucose levels involves the oxidation of glucose by molecular oxygen in the presence of glucose oxidase to produce gluconic acid and hydrogen peroxide. The electrochemical detection can be linked either to the oxygen depletion or to the peroxide generation.

In either case, a major disadvantage of this scheme is the limitation in oxygen availability. This limitation necessitates a pre-dilution to insure an adequate oxygen supply for the desired linear range of the concentrations expected to be encountered in the test fluid. In addition, whole blood measurement is especially difficult to measure accurately owing to the oxygen buffering capacity of hemoglobin.

Alternatively, the prior art has substituted a mediator for the oxygen in the chemical reaction, which is loaded into the membrane, or coating, on the sensing electrode to eliminate the need for pre-dilution. In the case where benzoquinone is selected as the mediator the reaction of glucose with benzoquinone in the presence of glucose oxidase produces gluconic acid and hydroquinone.

This alternative is subject to the disadvantage that the desired (and measured) reaction competes with the previously described reaction of glucose with molecular oxygen. Thus, samples with different oxygen concentrations can yield different current responses at the same glucose concentrations. This interference is most pronounced in the measurement of whole blood.

## Summary of the Invention

In accordance with one aspect of the present invention, there is provided in a single-use sensing device for use with a clinical chemistry analyzer capable of detecting the level, amperometrically, in a solution thereof, of a selected chemical species susceptible to dehydrogenation in the presence of a dehydrogenase enzyme therefor, which device comprises a carrier and at least two electrodes, the improvement wherein one of said electrodes is coated with a composition comprising methylene blue, $NAD^+$, a perfluorosulfonic acid polymer and an enzyme for the dehydrogenation of said selected chemical species.

In accordance with another aspect of the present invention, there is provided a method of determining the concentration in an aqueous solution, of a selected chemical species susceptible to dehydrogenation in the presence of a dehydrogenase enzyme therefor, which comprises wetting the outer surface of a coating on an electrode with said solution, permitting said solution to pass through said coating to contact said electrode and produce an electrical current between said electrode and another electrode, thereafter measuring the amperage of said current, said coating comprising, methylene blue, $NAD^+$, a perfluorosulfonic acid polymer and an enzyme for the dehydrogenation of said selected chemical species.

Preferably, the coating composition also contains a water soluble resinous polymer, such as polyvinylpyrrolidone (PVP) and an aqueous emulsion adhesive, such as a polyvinyl acetate latex adhesive.

For some dehydrogenation reactions it may be preferable to employ as a cofactor, in place of $NAD^+$, $NADP^+$ (nicotinamide adenine dinucleide phosphate), as is known in the art.

The invention is also applicable to chemical species which are susceptible to hydrogenation, rather than dehydrogenation, in the presence of a dehydrogenase enzyme, as for example, pyruvic acid. In such applications, the hydrogenated form of the cofactor, namely NADH or NADPH is used in the coating composition in place of $NAD^+$ or $NADP^+$.

In another embodiment of the invention, it may be used to detect the level of a dehydrogenase enzyme in a body fluid, or other aqueous solution. In such a case, the coating composition does not contain the dehydrogenase enzyme, but rather contains a species susceptible to hydrogenation or dehydrogenation by the enzyme to be measured in an amount in excess of the amount expected to be consumed by the dehydrogenase enzyme content of the body fluid to be tested.

In the system of this invention all of the components necessary to translate glucose content, for example, to amperage, are loaded into the coating membrane in a supply adequate for the expected linear range of glucose contents. Thus, pre-dilution of the samples is not necessary.

In addition, oxygen is not involved in the measurement; and oxygen concentration does not affect it.

The presence of methylene blue in the coating permits detection and measurement at lower applied voltage, thus eliminating interference from species which might be susceptible to oxidation at higher potentials.

The coated electrode of this invention responds rapidly, allowing accurate measurement in two minutes, or less.

Finally, the membrane composition is not soluble in aqueous solutions and retains its integrity for a long enough period to enable reliable measurement to be made. It is believed that the anionic perfluorosulfonic acid polymer binds the cationic methylene blue to the membrane.

The sensing electrode in this invention is the measurement anode, one of preferably two separate anodes transmitting electrons in parallel to a single cathode and to a ground. The other anode, called the "background anode" is maintained at a constant voltage relative to the cathode and to the ground. Thus, as is known in the art, changing conditions at the measurement anode caused by exposure to the test solution and the generation of electrons thereon will have only a negligible effect on the applied voltage between the anodes and the cathode.

Both the measurement anode and the background anode are preferably made of graphite. The cathode is preferably made of silver and has a silver chloride coating. It is only the measurement electrode that is coated with the aforementioned coating composition.

In the coating compositions in which the perfluorosulfonic acid polymer is the sole resinous constituent, the coating is too dense for rapid transmission of the calibrant liquid and the test liquid therethrough. Thus, while such compositions may be used in slower testing, they are not preferred for systems intended to provide rapid readouts.

In the preferred embodiment in which the coating composition also includes a water soluble resinous

polymer, the coating composition permits rapid transmission of the calibrant and test solutions therethrough.

The preferred composition also contains an aqueous emulsion adhesive, such as a polyvinyl acetate latex which forms a cross-linked structure on drying and thereby provides improved integrity to the composition.

## Brief Description of the Drawings

FIGURE 1 is an exploded perspective view of the carrier used in the system of the invention,

FIGURE 2 is an enlarged cross-sectional view of the measurement electrode of the invention,

FIGURE 3 is a graph illustrating the variation of current against time in two measurements in which a test solution containing a measured amount of glucose is released into a calibrant containing 5 millimolar glucose. In the first measurement, the test solution contains 2 millimolar glucose. In the second, it contains 20 millimolar glucose.

FIGURE 4 is a graph illustrating the variation of current against applied voltage for calibrants containing or not containing glucose and for coatings or membranes containing or not containing methylene blue.

FIGURE 5 is an enlarged fragmentary cross-sectional view of the upper portion of another embodiment of the measurement electrode of this invention.

## Detailed Description of the Invention

FIG. 1 shows the overall construction of sensing device 10 of this invention, including carrier 11, in the form of a rigid card, made of a tough, non-conducting plastic material such as an acrylonitrile-butadiene-styrene copolymer (ABS), and plate 12 covering one end thereof. Plate 12 is generally made of a transparent plastic material, but transparency is not essential.

At one end of carrier, under the plate, capillary passage 13 is S-shaped and is defined by the narrow space between the upper surface of carrier 11 and the lower surface of a substantially flat S-shaped cover. Capillary passage 13 runs between inlet end 14 and outlet end 16. At the inlet end, there is a raised portion 17 of the S-shaped capillary passage cover, called the "plow." It is the function of the plow to pierce the foil retaining the calibrant and the test solution, as hereinafter described, to permit these solutions, successively to enter inlet 14 of the capillary passage. Background anode 18, cathode 19 and measurement anode 21 are within the capillary passage, close to its inlet, and are each surrounded by a "moat" 33 as is shown in FIG. 2 for the measurement anode 21.

Cylindrical guide sleeve 22 is mounted in one corner of plate 18 over inlet 17. Multichamber cylinder 23 is mounted for rotation within sleeve 22 and has an interior wall 24, dividing the cylinder into calibrant chamber 26 which contains a calibrant liquid and sample chamber 27 which receives a sample of blood or other body fluid. The calibrant solution is factory sealed in chamber 26 and contains a known concentration of glucose, or other species to be measured by the sensor. Cap 28, which is connected by web 29 to cylinder 23, is placed over the upper end of cylinder 23 after sample chamber 27 has received the body fluid sample.

Measurement anode 21, shown in cross-section in FIG. 2, comprises graphite plug 31, preferably a cylinder cut from a graphite plug, extending through a perforation in the plastic material of carrier 11. A membrane, or coating 32 covers one surface of plug 31 and extends to moat 33 which surrounds the plug at a small distance therefrom. Conductive track 34 at the opposite surface of carrier 11 leads electrons generated at the measurement anode to a contact within the sensing device and ultimately to a microprocessor which provides the desired readout.

Background anode 18 is constructed similarly to measurement anode 21, except that it lacks a coating or membrane. Cathode 19 is a silver plug extending through carrier 11, coated at its upper surface with a thin layer of silver chloride.

Coating 32, as mentioned above, comprises at least glucose dehydrogenase (when glucose is the chemical species to be measured), methylene blue [3,7-Bis(dimethylamino)phenothiazin-5-ium chloride] and a perfluorosulfonic acid polymer.

Suitable perfluorosulfonic acid polymers which may be used are those having equivalent weights of at least about 900. Equivalent weights of at least about 1100 are preferred.

Perfluorosulfonic acid solutions are sold commercially by E.I. duPont de Nemours & Co. under the trademark Nafion® and can also be prepared by the procedure described by Martin et al. in Anal. Chem., Volume 54, 1639 (1982).

Preferably, the coating composition also contains a water soluble resinous polymer, such as polyvinylpyrrolidone. The presence of this material makes the coating or membrane, more easily penetratable by the test solution and enables faster readouts.

When polyvinylpyrrolidone (PVP) is the water soluble polymer, it is generally a polymer having an average molecular weight in excess of about 10,000, and preferably in excess of about 300,000.

Other water soluble polymers that may be used include polyethylene oxide, polyvinyl alcohol, hydroxyethyl cellulose, gum arabic and alginic acid.

In addition, the preferred coating composition also contains an aqueous emulsion adhesive, such as a polyvinyl acetate latex adhesive. This material cross links on drying to cure and thereby provide additional integrity to the dried coating, make it less likely to disintegrate when wetted by the test solution.

Other aqueous emulsion adhesives that may be used include latex polymers of acrylate and methacrylate esters.

A typical coating composition sufficient for the preparation of measurement anodes for about 1000 disposable carriers contains from about 2000 to about 3500 units of glucose dehydrogenase (or from about 0.04 to about 0.07 grams thereof based on 50 units/mg activity), from about 0.2 to about 0.5 grams of nicotinamide adenine dinucleide, from about 0.01 to about 0.03 grams of methylene blue and from about 1 to about 2 milliliters of perfluorosulfonic acid polymer as a solution in methanol containing 1.25 weight percent of the polymer.

The coating composition is applied to the upper surface of the measurment electrode and then allowed to dry. The moat which surrounds the measurement electrode provides a sharp surface at the edge of the electrode and thereby provides, by surface tension, a sharp boundary for the coating composition, limiting its spread to precisely the area of the electrode.

Although graphite has an oily, or hydrophobic, surface, it has been found, surprisingly, that the aqueous coating composition is not repelled thereby and that the coating, after drying is adherent thereto.

When the coating is dried, it contains, typically, from about 4000 to about 8000 units of glucose dehydrogenase per gram of coating (or from about 8 to about 16 weight percent thereof), from about 75 to about 87 weight percent of $NAD^+$, from about 2 to about 6 weight percent of methylene blue and from about 2 to about 4 weight percent of perfluorosulfonic acid polymer. In addition, the coating contains from zero to about 7 weight percent of a water soluble polymer, and preferably from about 2 to about 4 weight percent thereof and from zero to about 5 weight percent of a cured emulsion adhesive and preferably from about 1 to about 3 weight percent thereof.

The coating, after drying, is generally between about 2 and about 4 mils in thickness, and preferably between about 3 and about 3.5 mils.

In a specific embodiment, a coating composition for about 1000 electrodes comprises:

1) glucose dehydrogenase 2235 u.
2) nicotinamide adenine dinucleide, sodium salt 0.298 g.
3) methylene blue 0.175 g.
4) polyvinylpyrrolidone (1% in water) 1.300 g.
5) polyvinyl acetate latex (2.1% in water) 0.388 g.
6) perfluorosulfonic acid polymer (1.25% in methanol) 1.313 ml.

The coating composition preferably contains about 500 to about 1000 units per milliliter of glucose dehydrogenase, from about 9 to about 15 weight percent of $NAD^+$, from about 0.3 to about 0.6 weight percent of perfluorosulfonic acid polymer, from about 0.4 to about 0.9 weight percent of methylene blue and from about 0.1 to about 0.6 weight percent of polyvinyl acetate latex. The remainder of the composition is solvent, primarily water.

The glucose dehydrogenase, the $NAD^+$ and the methylene blue are first dissolved in the aqueous PVP solution, with stirring. Then the polyvinyl acetate latex is added with stirring until completely blended. While the composition is being stirred, the perfluorosulfonic acid polymer solution is added dropwise. Slow addition of this component, over a period of 1-2 minutes is necessary to produce a fine dispersion of the polymer within the coating composition.

The appearance of the coating composition at this stage is generally very dark blue with dispersed, fine black particles.

The properties of the measurement electrode in operation are shown in FIGURES 3 and 4. FIGURE 3 relates to two tests. It shows, in a first test, the amperage at the electrode varied with time as it is exposed, first to a calibrant solution containing 5 millimolar glucose and then (after 120 seconds) to a test solution made to contain 2 millimolar glucose. The second test is similar, except that the test solution is made to contain 20 millimolar glucose.

Reading the left curves of FIGURE 3 from left to right shows that the electrode initially generates a small amount of current (about 1 milliampere) by reason of random and unknown electrical "noise" and that the current rises within about 60 seconds to a value close to 3 milliamperes as the calibrant solution reaches the electrode.

When the test solution is released, after about 120 seconds, there is a momentary slight rise in the amperage due to the mixing effect and then a steady drop in the amperage as the test solution dilutes the calibrant solution and lowers its glucose content.

In contrast, the right curve also shows and increase in amperage during the first 60 seconds as the calibrant solution reaches the electrode. This is followed by a large increase in amperage after 120 seconds when the second test solution is released and starts to raise the glucose content of the solution as it blends into and replaces the calibrant solution. After an interval of about 180 seconds, the peak amperage of about 10 milliamperes is obtained; and it is at this time that the amperage reading is taken by the device and correlated with the amperage reading for the calibrant solution to provide the desired reading of the glucose content of the test solution.

For the particular system used in the FIGURE 3 tests, made with the aforesaid specific coating composition, the optimum time for a reading of the test solution amperage is about 180 seconds after the release of the calibrant solution and about 60 seconds after the release of the test solutions. With other coating compositions the optimum reading time will vary from as little as 2 minutes after release of the calibrant solution to as much as twenty minutes or more.

FIGURE 4 contains four separate curves and illustrates the variation of measured current at varying imposed

potential for a calibrant containing no glucose (curves A and B) and for a calibrant containing 5 millimolar glucose (curves C and D). Curves A and C represent a system in which the anode coating contains no methylene blue. Curves B and D represent a system where the coating contains methylene blue in the amount set forth in the foregoing specific embodiment.

As may be seen, there is a far greater difference between Curves B and D at lower voltages than between Curves A and B; and the level of current in Curves A and B (indicating current not attributable to glucose) is unacceptably high at the higher voltages. The curves indicate that the presence of methylene blue in the coating composition provides a test of far greater sensitivity and accuracy than can be obtained in its absence. Optimum results are obtained at an imposed potential between about 0.2 and about 0.4 volts.

FIG. 5 illustrates another embodiment of this invention which is useful where extreme accuracy is desired at a sacrifice of rapidity of readout. In FIG. 5, elements similar to those of FIG. 2 bear similar reference numerals; and it may be seen that reactant-bearing coating 32 in this embodiment is not in direct contact with the graphite anode nor with the applied test solution. Rather, the reactant bearing coating is sandwiched between two layers 41 and 42 of perfluorosulfonic acid polymer. The resistance of the perfluorosulfonic acid polymer layers to disintegration in aqueous media slows the penetration of the test fluid to the anode and provides integrity to the coating assembly so that it retains cohesiveness for the longer contact period required.

The embodiment of FIG. 5 is particularly useful for determinations, such as the ethyl alcohol level in blood, in which extreme precision must be assured and where rapidity of readout is of secondary importance.

The Table hereinbelow provides a comparison of the performance characteristics of the system of the present invention in comparison to a commercially available glucose amperometric measurement system utilizing the glucose oxidase reaction to generate current.

TABLE

| | Prior Art System | System of this Invention |
|---|---|---|
| Precision | | |
| with plasma | 3-8% | 3-5% |
| with whole blood | 6-10% | 3-5% |
| Lowest Detection Level with whole blood | 3 millimoles/liter | under 1 millimole/liter |
| Highest Detection Level | 20 millimoles/liter | 30-35 millimoles/liter |
| Oxygen Interference | severe, oxygen depresses signal | none |
| Temperature effect with whole blood | severe | only 1/4 of oxidase system |
| Accuracy | | |
| with plasma | ± 1 millimole/liter | under 1 millimole/liter |
| with whole blood | ± 1.5 millimoles/liter | under 1 millimole/liter |

In operation, the operator first inserts a body sample into chamber 27, then seals the chambers with cap 28 and inserts the card assembly into the appropriate slot in the sensing device. He then rotates cap 28 and cylinder 23 by 90° from a start position, thereby rupturing a foil seal at the bottom of chamber 26 through piercing by plow 17 and permitting calibrant liquid from chamber 26 to flow into capillary passage 13 where it contacts all three electrodes 18, 19 and 21. When the flow of calibrant liquid stops, due to reduced hydraulic head and capillary action, the calibrant test reading is made by the analyzer in response to the glucose level of the calibrant. Once this reading has been made (generally after about 120 seconds), the operator rotates cap 28 and cylinder 23 once more, this time by 180°, to a sample test position at which a foil seal at the bottom of chamber 27 is pierced by plow 17 and the test liquid flows into inlet 14 of capillary passage 13.

The test fluid, at this point, displaces the calibrant fluid in the portion of capillary passage 13 which contains electrodes 18, 19 and 21. The flow of the test fluid into the capillary passage also stops, due to decreasing hydraulic head and capillary action; and final measurements are made by the analyzer. Based upon sensor readings when the calibrant liquid is in contact with measurement anode 21 and when the test liquid is in contact with measurement anode 21, the analyzer derives the concentration of glucose in the sample fluid.

The invention has been described, principally, with respect to the determination of glucose levels. However, it may be used to detect other chemical species which are dehydrogenatable or hydrogenatable in the presence of an appropriate dehydrogenase enzyme, including ethanol, lactate, pyruvate, cholesterol, malate, glycerol and glutamate.

Although the present invention has been described with reference to preferred embodiments, those skilled in the art will recognize that changes may be made in form and detail without departing from the teachings and scope of the invention.

**Claims**

1. In a single-use sensing device for use with a clinical chemistry analyzer capable of detecting, the level, amperometrically, in a solution thereof, of a selected chemical species susceptible to dehydrogenation in the presence of a dehydrogenase enzyme therefor, which device comprises a carrier and at least two electrodes, the improvement wherein one of said electrodes is coated with a composition comprising methylene blue, a cofactor of the group consisting of $NAD^+$ and $NADP^+$, a perfluoro-sulfonic acid polymer and an enzyme for the dehydrogenation of said selected chemical species.

2. In a single-use sensing device for use with a clinical chemistry analyzer capable of detecting, the level, amperometrically, in a solution thereof, of a selected chemical species susceptible to hydrogenation in the presence of a dehydrogenase enzyme therefor, which device comprises a carrier and at least two electrodes, the improvement wherein one of said electrodes is coated with a composition comprising methylene blue, a cofactor of the group consisting of NADH an NADPH, a perfluorosulfonic acid polymer and an enzyme for the dehyrogenation of said selected chemical species.

3. In a single-use sensing device for use with a clinical chemistry analyzer capable of detecting, the level, amperometrically, in a solution thereof, of a dehydrogenase enzyme therefor, which device comprises a carrier and at least two electrodes, the improvement wherein one of said electrodes is coated with a composition comprising methylene blue, a cofactor of the group consisting of $NAD^+$ and $NADP^+$, a perfluoro-sulfonic acid polymer and a selected chemical species susceptible to susceptible to hydrogenation in the presence of said dehydrogenase enzyme enzyme.

4. The sensing device of claim 1 wherein said enzyme is glucose dehydrogenase.

5. The sensing device of any one of claims 1, 2 and 3 wherein said coating composition contains a water soluble resinous polymer.

6. The sensing device of claim 5 wherein said water soluble resinous polymer comprises polyvinylpyrrolidone.

7. The sensing device of any one of claims 1, 2 and 3 wherein said coating composition contains an aqueous emulsion adhesive.

8. The sensing device of claim 7 wherein said adhesive comprises a polyvinyl acetate latex.

9. The sensing device of any one of claims 1, 2 and 3 wherein said coating is from about 2 to about 4 mils in thickness.

10. The sensing device of claim 4 wherein said coating contains from about 4000 to about 8000 units of glucose dehydrogenase per gram and contains from about 75 to about 87 weight percent of $NAD^+$, from about 2 to about 4 weight percent of perfluorosulfonic acid polymer and from about 3 to about 6 weight percent of methylene blue.

11. The sensing device of claim 10 wherein said coating contains, in addition, from about 2 to about 4 weight percent of polyvinylpyrrolidone and from about 1 to about 3 weight percent of cured polyvinyl acetate.

12. The method of determining the concentration, in an aqueous solution, of a selected chemical species susceptible to dehydrogenation in the presence of a dehydrogenase enzyme therefor, which comprises wetting the outer surface of a coating on an electrode with said solution, permitting said solution to pass through said coating to contact said electrode and produce an electrical current between said electrode and another electrode and thereafter measuring the amperage of said current, said coating comprising methylene blue, a cofactor of the group consisting of $NAD^+$, and $NADP^+$, a perfluorosulfonic acid polymer and an enzyme for the dehydrogenation of said selected chemical species.

13. The method of determining the concentration, in an aqueous solution, of a selected chemical species susceptible to hydrogenation in the presence of a dehydrogenase enzyme therefor, which comprises wetting the outer surface of coating on an electrode with said solution, permitting said solution to pass through said coating to contact said electrode and produce an electrical current between said electrode and another electrode and thereafter measuring the amperage of said current, said coating comprising methylene blue, a cofactor of the group consisting of NADPH and NADPH, a perfluorosulfonic acid polymer and an enzyme for the dehydrogenation of said selected chemical species.

14. The method of determining the concentration, in an aqueous solution, of a dehydrogenase enzyme chemical species susceptible to dehydrogenation in the presence of a dehydrogenase enzyme therefor, which comprises wetting the outer surface of a coating on an electrode with said solution, permitting said

solution to pass through said coating to contact said electrode and produce an electrical current between said electrode and another electrode and thereafter measuring the amperage of said current, said coating comprising methylene blue, a cofactor of the group consisting of $NAD^+$, and $NADP^+$, a perfluorosulfonic acid polymer and a said selected chemical species susceptible to dehydrogenation in the presence of said enzyme.

15. The method of any one of claims 12, 13 and 14 wherein said outer surface is wetted with a calibration solution prior to being wetted with said solution of said selected chemical species.

16. The method of claim 12 wherein said selected chemical species is glucose and said dehydrogenase enzyme is glucose dehydrogenase.

17. The method of claim 12 wherein said selected chemical species is ethanol and said dehydrogenase enzyme is alcohol dehydrogenase.

18. The method of any one of claims 12, 13 and 14 wherein said coating contains, in addition, a water soluble resinous polymer and a cured emulsion adhesive.

19. The method of claim 18 wherein said water soluble resinous polymer is polyvinylpyrrolidone and said cured emulsion adhesive is cured polyvinyl acetate.

20. The method of claim 19 wherein said coating comprises from about 4000 to about 8000 units per gram of glucose dehydrogenase, from about 75 to about 87 weight percent of $NAD^+$, from about 3 to about 6 weight percent of perfluorosulfonic acid polymer and from about 2 to about 4 weight percent of methylene blue.

21. The method of any one of claims 12, 13 and 14 wherein said amperage is measured at a superimposed potential.

22. The method of claim 21 wherein said superimposed potential is between about 0.2 and about 0.4 volts.

23. An electrode coating composition comprising a cofactor of the group consisting of $NAD^+$, NADH, $NADP^+$, $NADP^+$ and NADPH, a perfluorosulfonic acid polymer, methylene blue and a member of the group consisting of dehydrogenase enzymes and chemical species susceptible to hydrogenation or dehydrogenation in the presence of a dehydrogenase enzyme.

24. The composition of claim 23 wherein said enzyme is glucose dehydrogenase.

25. The composition of claim 23 wherein said coating composition contains, in addition, polyvinyl pyrrolidone and a polyvinyl acetate latex.

26. The composition of claim 23 wherein said coating composition contains from about 500 to about 1000 units per milliliter of glucose dehydrogenase, from about 9 to about 15 weight percent of $NAD^+$, from about 0.3 to about 0.6 weight percent of perfluorosulfonic acid polymer, from about 0.4 to about 0.9 weight percent of methylene blue, from about 0.1 to about 0.6 weight percent of polyvinyl acetate latex.

27. An electrode for a sensing device comprising an electrically conductive body having a coating thereon comprising a cofactor of the group consisting of $NAD^+$, NADH, $NADP^+$, NADPH, a perfluorosulfonic acid polymer, polyvinylpyrrolidone, cured polyvinyl acetate latex and a member of the group consisting of dehydrogenase enzymes and chemical species susceptible to hydrogenation or dehydrogenation in the presence of a dehydrogenase enzyme.

28. The electrode of claim 27 wherein said electrically conductive body is graphite.

29. The electrode of claim 27 wherein said coating comprises from about 75 to about 87 weight percent of $NAD^+$, from about 2 to about 4 weight percent of perfluorosulfonic acid polymer, from about 1 to about 3 weight percent of cured polyvinyl acetate latex and from about 4000 to about 8000 units per gram of coating of glucose dehydrogenase.

30. The electrode of claim 27 wherein said coating is a single layer coating.

31. The electrode of claim 27 wherein said coating comprises a multilayer coating in which a layer containing the components set forth in claim 23 is sandwiched between two layers consisting essentially of perfluorosulfonic acid polymer.

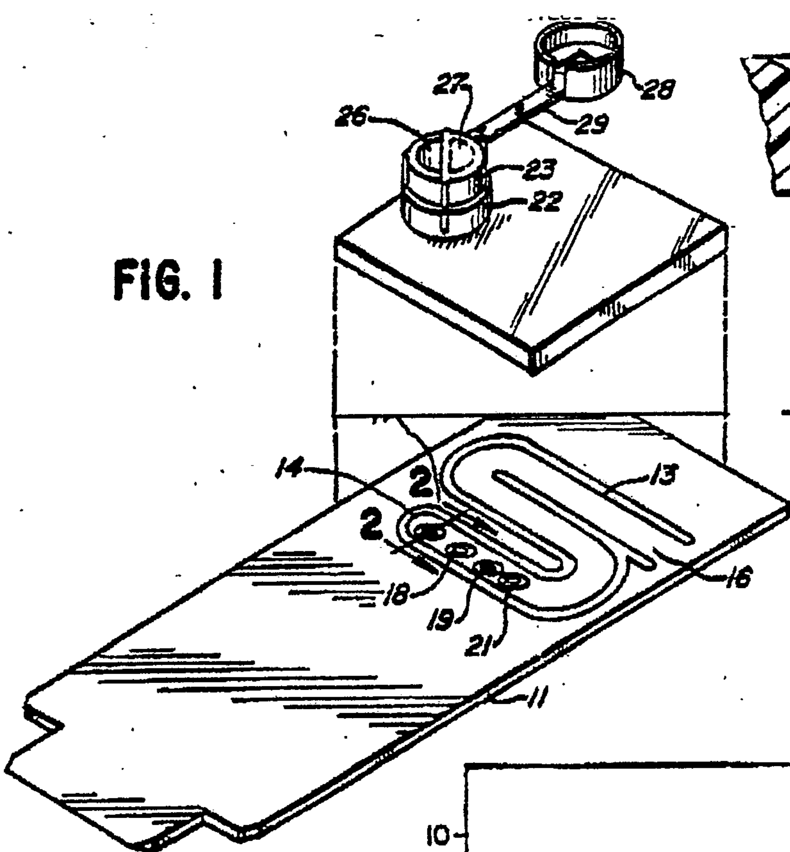


FIG. 1

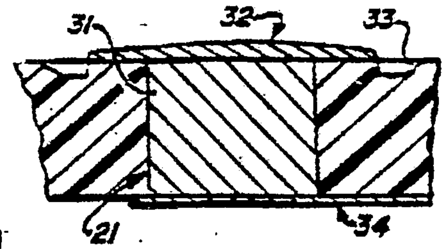


FIG. 2

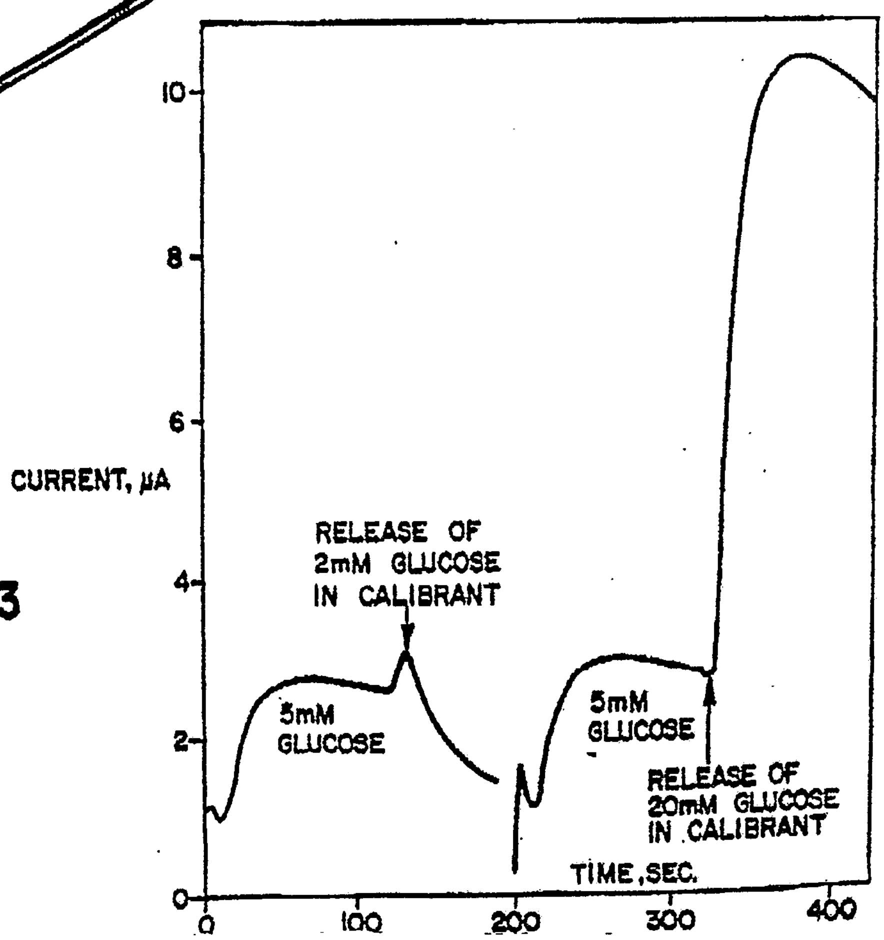


FIG. 3

FIG. 4

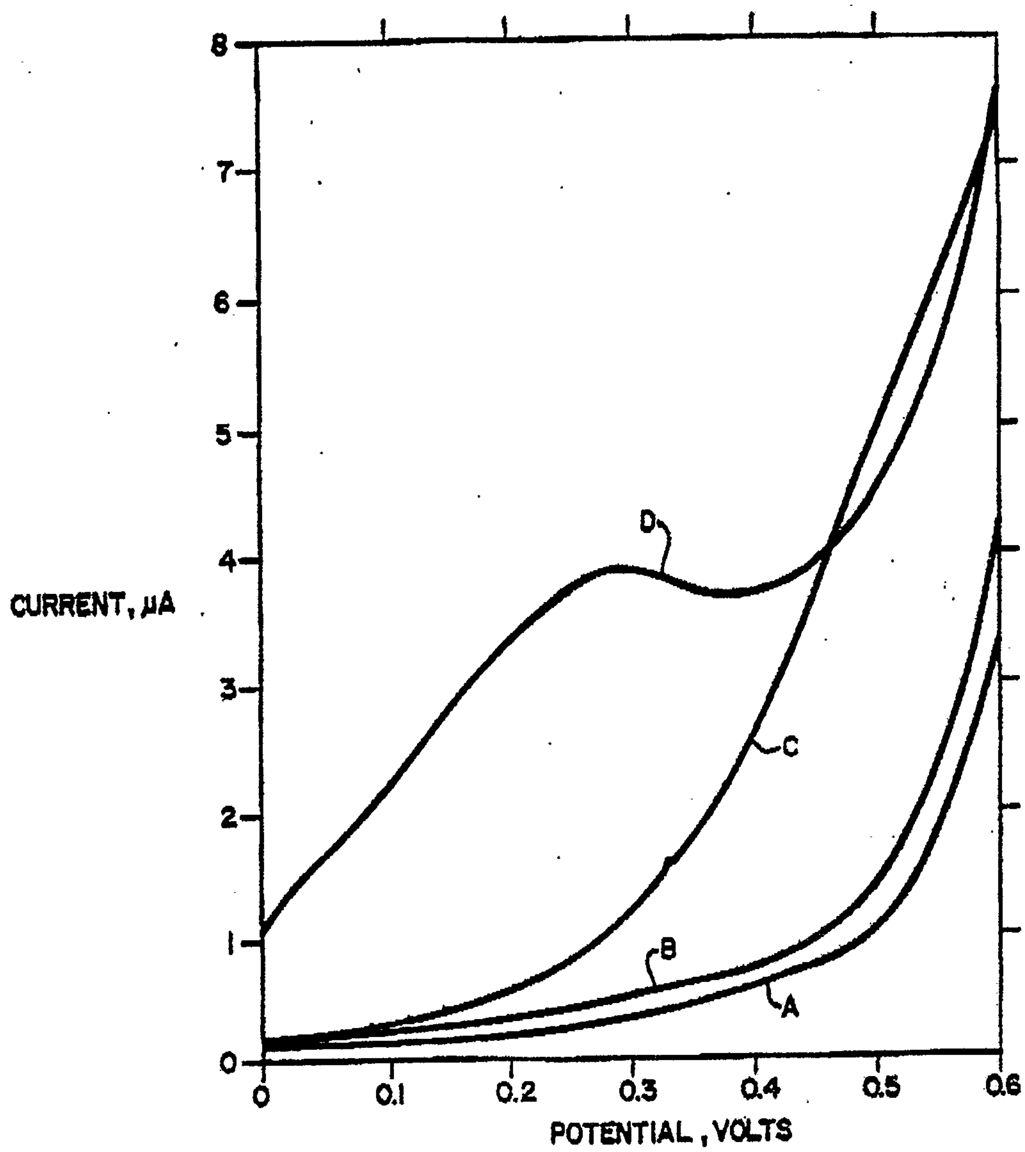
8
7
6
5
4
3
2
1
0
CURRENT, µA
D
C
B
A
0
0.1
0.2
0.3
0.4
0.5
0.6
POTENTIAL, VOLTS

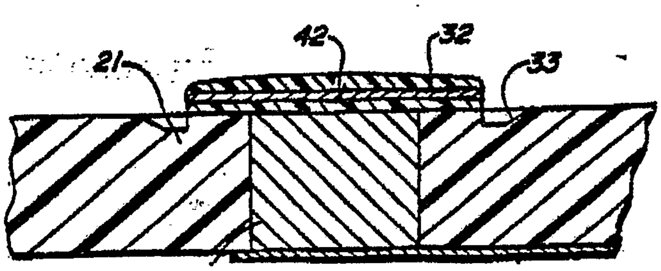
21
42
32
33

Fig 5